# EUROPEAN PATENT SPECIFICATION

(11) **EP 0 334 301 B1**
(45) Date of publication and mention of the grant of the patent: **23.12.1998**
(21) Application number: 89105059.3
(22) Date of filing: 21.03.1989
(51) Int. Cl.: C12N 7/00, C12N 15/00, A61K 35/76, G01N 33/569

(54) **Recombinant retroviruses**
Rekombinante Retroviren
Rétrovirus recombinants

(30) Priority: 21.03.1988 US 170515
(43) Date of publication of application: 27.09.1989
(60) Divisional application: 95115441.8
(73) Proprietor: CHIRON CORPORATION, Emeryville, California 94608 (US)
(72) Inventor: Gruber, Harry E., San Diego California 92130 (US); Jolly, Douglas J., LaJolla California 92037 (US); Respess, James G., San Diego California 92109 (US); Laikind, Paul K., San Diego California 92130 (US)
(74) Representative: Goldin, Douglas Michael

(56) References cited:
- EP-A- 0 243 204
- WO-A-90/01870
- FR-A- 2 559 159
- FR-A- 2 606 030
- SCIENCE, vol. 229, 26th July 1985, pages 345-352; J.G. IZANT et al.: "Constitutive and conditional suppression of exogenous and endogenous genes by anti-sense RNA"
- NATURE, vol. 318, 5th December 1985, page 414; R. TELLIER et al.: "New strategies for AIDS therapy and prophylaxis"
- PROCEEDINGS OF THE NATIONAL ACADEMY OF SCIENCES OF THE USA, vol. 83, July 1986, pages 4794-4798; J.T. HOLT et al.: "Inducible production of c-fos antisense RNA inhibits 3T3 cell proliferation"
- SCIENCE, vol. 239, 8th January 1988, pages 184-187; B.K. FELBER et al.: "A quantitative bioassay for HIV-1 based on trans-activation"
- BIOTECHNOLOGY, vol. 3, no. 8, August 1985, pages 689-693, New York, US; D. McCORMICK: "Human gene therapy: The first round"
- NATURE, vol. 331, no. 6151, 7th January 1988, pages 78-81, London, GB; R.E. HUSSEY et al.: "A soluble CD4 protein selectively inhibits HIV replication and syncytium formation"

## Description

### Technical Field

The present invention relates generally to retroviruses, and more specifically, to recombinant retroviruses which are capable of delivering vector constructs to susceptible target cells. These vector constructs are typically designed to express desired proteins in target cells, for example, proteins which stimulate immunogenic activity or which are conditionally active in defined cellular environments.

### Background of the Invention

Although bacterial diseases are, in general, easily treatable with antibiotics, very few effective treatments or prophylactic measures exist for many viral, cancerous, and other nonbacterial diseases, including genetic diseases. Traditional attempts to treat these diseases have employed the use of chemical drugs. In general, these drugs have lacked specificity, exhibited high overall toxicity, and thus have been therapeutically ineffective.

Another classic technique for treating a number of nonbacterial diseases involves the elicitation of an immune response to a pathogenic agent, such as a virus, through the administration of a noninfectious form of the agent, such as a killed virus, thereby providing antigens from the pathogenic agent which would act an an immunostimulant.

A more recent approach for treating viral diseases, such as acquired immunodeficiency syndrome (AIDS) and related disorders, involves blocking receptors on cells susceptible to infection by HIV from receiving or forming a complex with viral envelope proteins. For example, Lifson et al. (Science 232:1123-1127, 1986) demonstrated that antibodies to CD4 (T4) receptors inhibited cell fusion (syncytia) between infected and noninfected CD4 presenting cells in vitro. A similar CD4 blocking effect using monoclonal antibodies has been suggested by McDougal et al. (Science 231:382-385, 1986). Alternatively, Pert et al. (Proc. Natl. Acad. Sci. USA 83:9254-9258, 1986) have reported the use of synthetic peptides to bind T4 receptors and block HIV infection of human T-cells, while Lifson et al. (J. Exp. Med. 164:2101, 1986) have reported blocking both syncytia and virus/T4 cell fusion by using a lectin which interacts with a viral envelope glycoprotein, thereby blocking it from being received by CD4 receptors.

A fourth, recently suggested technique for inhibiting a pathogenic agent, such as a virus, which transcribes RNA is to provide antisense RNA which complements at least a portion of the transcribed RNA, and binds thereto, so as to inhibit translation (To et al., Mol. Cell. Biol. 6:758, 1986).

However, a major shortcoming of the techniques described above is that they do not readily lend themselves to control as to the time, location or extent to which the drug, antigen, blocking agent or antisense RNA are utilized. In particular, since the above techniques require exogenous application of the treatment agent (i.e., exogenous to the sample in an in vitro situation), they are not directly responsive to the presence of the pathogenic agent. For example, it may be desirable to have an immunostimulant expressed in increased amounts immediately following infection by the pathogenic agent. In addition, in the case of antisense RNA, large amounts would be required for useful therapy in an animal, which under current techniques would be administered without regard to the location at which it is actually needed, that is, at the cells infected by the pathogenic agent.

As an alternative to exogenous application, techniques have been suggested for producing treatment agents endogenously. More specifically, proteins expressed from viral vectors based on DNA viruses, such as adenovirus, simian virus 40, bovine papilloma, and vaccinia viruses, have been investigated. By way of example, Panicali et al. (Proc. Natl. Acad. Sci. USA 80:5364, 1983) introduced influenza virus hemagglutinin and hepatitis B surface antigens into the vaccinia genome and infected animals with the virus particles produced from such recombinant genes. Following infection, the animals acquired immunity to both the vaccinia virus and the hepatitis B antigen.

However, a number of difficulties have been experienced to date with viral vectors based on DNA viruses. These difficulties include (a) the production of other viral proteins which may lead to pathogenesis or the suppression of the desired protein; (b) the capacity of the vector to uncontrollably replicate in the host, and the pathogenic effect of such uncontrolled replication; (c) the presence of wild-type virus which may lead to viremia; and (d) the transitory nature of expression in these systems. These difficulties have virtually precluded the use of viral vectors based on DNA viruses in the treatment of viral, cancerous, and other nonbacterial diseases, including genetic diseases.

Due to the nontransitory nature of their expression in infected target cells, retroviruses have been suggested as a useful vehicle for the treatment of genetic diseases (for example, see F. Ledley, The Journal of Pediatrics 110:1, 1987). However, in view of a number of problems, the use of retroviruses in the treatment of genetic diseases has not been attempted. Such problems relate to (a) the apparent need to infect a large number of cells in inaccessible tissues (e.g., brain); (b) the need to cause these vectors to express in a very controlled and permanent fashion; (c) the lack of cloned genes; (d) the irreversible damage to tissue and organs due to metabolic abnormalities; and (e) the availability of other partially effective therapies in certain instances.

In addition to genetic diseases, other researchers have contemplated using retroviral vectors to treat nongenetic diseases (see, for example, EP 243,204 - Cetus Corporation; Sanford, J. Theor. Biol.130:469, 1988; Tellier et al., Nature 318:414, 1985; and Bolognesi et al., Cancer Res. 45:4700, 1985).

Tellier et al. suggested protecting T-cell clones by apparently infecting stem cells with "defective" HIV having a genome which could express antisense RNA to HIV RNA. Bolognesi et al. have suggested the concept of generating a nonvirulent HIV strain to infect stem cells so that T4 cells generated therefrom would carry interfering, nonvirulent forms of virus and thereby protect those cells from infection by virulent HIV. However, it would appear that the "attenuated" or "defective" HIV viruses used in both of the foregoing papers could reproduce (i.e., are not replication defective) such that the resulting viruses could infect other cells, with the possibility of an increased risk of recombination with previously present HIV or other sequences, leading to loss of attenuation. Non-nonreplicative forms would necessitate a defective helper or packaging line for HIV. However, since the control of HIV gene expression is complex, such cells have to date not been constructed. Furthermore, as the infecting attenuated or defective virus is not chimeric (a "nonchimeric" retrovirus being one with substantially all of its vector from the same retrovirus species), even if they were made replication defective, for example, by deletion from their genomes of an essential element, there still exists a significant possibility for recombination within the host cells with resultant production of infectious viral particles.

Although Sanford (J. Theor. Biol. 130:469, 1988) has also proposed using a genetic cure for HIV, he notes that due to the potential that exists for creating novel virulent viruses via genetic recombination between natural AIDS virus and therapeutic retroviral vectors carrying anti-HIV genes, retroviral gene therapy for AIDS may not be practical. Similarly, while McCormick & Kriegler (EP 243,204 A2) have proposed using retroviral vectors to deliver genes for proteins, such as tumor necrosis factor (TNF), the techniques they describe suffer from a number of disadvantages.

### Summary of the Invention

Briefly stated, the present invention is directed, in part, toward methods for stimulating a specific cell-mediated immune response through the use of replication-defective recombinant retroviruses.

More specifically, within one aspect of the present invention, there is provided a replication-defective recombinant retrovirus capable of infecting human cells carrying a vector construct capable of preventing, inhibiting, stabilizing or reversing infectious, cancerous or auto-immune diseases,
wherein the vector construct directs the expression of a disease antigen which is from a pathogenic agent or is a cancer antigen or modified form thereof in target cells infected with the retrovirus,
said antigen or modified form thereof being capable of stimulating a cell-mediated immune response within a human.

Where an immune response is to be stimulated to a pathogenic antigen, the recombinant retrovirus is preferably designed to express a modified form of the antigen which will stimulate a cell-mediated immune response and which has reduced pathogenicity relative to the native antigen.

In the particular case of disease caused by HIV infection, where immunostimulation is desired, the antigen generated from the recombinant retroviral genome is of a form which will elicit either or both an HLA class I- or class II-restricted immune response. In the case of HIV envelope antigen, for example, the antigen is preferably selected from gp 160, gp 120, and gp 41, which have been modified to reduce their pathogenicity. In particular, the antigen selected is modified to reduce the possibility of syncytia. Antigens from other HIV genes, such as gag, pol, vif, nef, etc., may also provide protection in particular cases.

Specificity for the pathogenic condition can also be attained or further enhanced through targeting of the entry of the recombinant retroviruses to the cells affected by the pathogenic condition. It should be understood in the foregoing discussion, and throughout this application, that when reference is made to the viral construct "expressing" or "producing" any substance in a cell, or the like, this in fact refers to the action of the resulting provirus following reverse transcription of the viral RNA in the cell.

Regardless of the means by which the recombinant retrovirus exerts its immunogenic action as described above, the retroviral genome will be "replication defective" (i.e., incapable of reproducing in cells infected with it). Thus, there will be only a single stage of infection in either an in vitro or in vivo application, thereby substantially reducing the possibility of insertional mutagenesis. Preferably, to assist in this end, the recombinant retrovirus lacks at least one of the gag, pol, or env genes. Further, the recombinant viral vector is preferably chimeric (that is, the gene which is to produce the desired result is from a different source than the remainder of the retrovirus). A chimeric construction further reduces the possibility of recombination events within cells infected with the recombinant retrovirus, which could produce a genome that can generate viral particles.

Within another aspect, the present invention also provides a method for producing such recombinant retroviruses of the invention in which the retroviral genome is packaged in a capsid and envelope, preferably through the use of a packaging cell. The packaging cells are provided with viral protein-coding sequences, preferably in the form of two plasmids, which produce all proteins necessary for production of viable retroviral particles, an RNA viral construct which will carry the desired gene along with a packaging signal which will direct packaging of the RNA into the retroviral particles.

There are now discussed below a number of techniques for producing recombinant retroviruses which can facilitate:
i) the production of higher titres from packaging cells;
ii) packaging of vector constructs by means not involving the use of packaging cells;
iii) the production of recombinant retroviruses which can be targeted for preselected cell lines; and
iv) the integration of the proviral construct into a preselected site or sites in a cell's genome.

One technique for producing higher titres from packaging cells takes advantage of the discovery that of the many factors which can limit titre from a packaging cell, one of the most limiting is the level of expression of the packaging proteins, namely, the gag, pol, and env proteins, as well as the level of expression of the retroviral vector RNA from the proviral vector. This technique allows the selection of packaging cells which have higher levels of expression (i.e., produce higher concentrations) of the foregoing packaging proteins and vector construct RNA. More specifically, this technique allows selection of packaging cells which produce high levels of what is referred to herein as a "primary agent," which is either a packaging protein (e.g., gag, pol, or env proteins) or a gene of interest to be carried into the genome of target cells (typically a vector construct). This is accomplished by providing in packaging cells a genome carrying a gene (the "primary gene") which expresses the primary agent in the packaging cells, along with a selectable gene, preferably downstream from the primary gene. The selectable gene expresses a selectable protein in the packaging cells, preferably one which conveys resistance to an otherwise cytotoxic drug. The cells are then exposed to a selecting agent, preferably the cytotoxic drug, which enables identification of those cells which express the selectable protein at a critical level (i.e., in the case of a cytotoxic drug, by killing those cells which do not produce a level of resistance protein required for survival).

Preferably, in the technique briefly described above, the expression of both the selectable and primary genes is controlled by the same promoter. In this regard, it may be preferable to utilize a retroviral 5' LTR. In order to maximize titre of a recombinant retrovirus from packaging cells, this technique is first used to select packaging cells expressing high levels of all the required packaging proteins, and then is used to select which of these cells, following transfection with the desired proviral construct, produce the highest titres of the recombinant retrovirus.

Techniques are also provided for packaging of vector constructs by means not involving the use of packaging cells. These techniques make use of DNA viruses such as baculovirus, adenovirus, or vaccinia virus, preferably adenovirus. These viruses are known to express relatively high levels of proteins from exogenous genes provided therein. For such DNA virus vectors, recombinant DNA viruses can be produced by in vivo recombination in tissue culture between viral DNA and plasmids carrying retroviral or retroviral vector genes. The resultant DNA viral vectors carrying either sequences coding for retroviral proteins or for retroviral vector RNA are purified into high titre stocks. Alternatively, the constructs can be constructed in vitro and subsequently transfected into cells which provide in trans viral functions missing from the DNA vectors. Regardless of the method of production, high titre (10⁷ to 10¹¹ units/ml) stocks can be prepared that will, upon infection of susceptible cells, cause high level expression of retroviral proteins (such as gag, pol, and env) or RNA retroviral vector genomes, or both. Infection of cells in culture with these stocks, singly or in combination, will lead to high-level production of retroviral vectors, if the stocks carry the viral protein and viral vector genes. This technique, when used with adenovirus or other mammalian vectors, allows the use of primary cells (e.g., from tissue explants or cells such as WI38 used in production of vaccines) to produce recombinant retroviral vectors.

In an alternative to the foregoing technique, recombinant retroviruses are produced by first generating the gag/pol and env proteins from a cell line infected with the appropriate recombinant DNA virus in a manner similar to the preceding techniques, except that the cell line is not infected with a DNA virus carrying the vector construct. Subsequently, the proteins are purified and contacted with the desired viral vector RNA made in vitro, transfer RNA (tRNA), liposomes, and a cell extract to process the env protein into the liposomes, such that recombinant retroviruses carrying the viral vector RNA are produced. Within this technique, it may be necessary to process the env protein into the liposomes prior to contacting them with the remainder of the foregoing mixture. The gag/pol and env proteins may also be made after plasmid mediated transfection in eukaryotic cells, in yeast, or in bacteria.

The technique for producing recombinant retroviruses which can be targeted for preselected cell lines utilizes recombinant retroviruses having an env gene comprised of a cytoplasmic segment of a first retroviral phenotype, and an extracellular binding segment exogenous to the first retroviral phenotype. The binding segment is from a second viral phenotype or from another protein with desired binding properties which is selected to be expressed as a peptide which will bind to the desired target.

Techniques for integrating a retroviral genome at a specific site in the DNA of a target cell involve the use of homologous recombination, or alternatively, the use of a modified inteqrase enzyme which will recognize a specific site on the target cell genome. Such site-specific insertion allows genes to be inserted at sites on the target cells' DNA, which will minimize the chances of insertional mutagenesis, minimize interference from other sequences on the DNA, and allow insertion of sequences at specific target sites so as to reduce or eliminate the expression of an undesirable gene (such as a viral gene) in the DNA of the target cell.

It will be appreciated that any of the above-described techniques may be used independently of the others in particular situations, or can be used in conjunction with one or more of the remainder of the techniques.

These and other aspects of the present invention will become evident upon reference to the following detailed description and attached drawings.

### Brief Description of the Drawings

Figure 1 depicts three different families of vectors used to produce HIV env and which may or may not have the selectable SV-Neo cassette inserted.

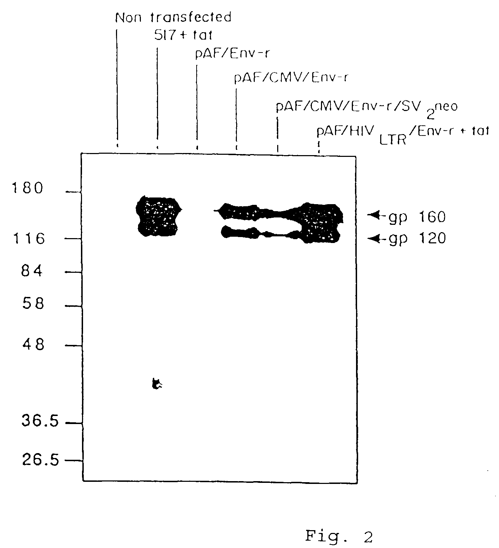

Figure 2 illustrates the HIV env expression levels seen in polyacrylamide gel electrophoresis of HIV env specific radioimmune precipitations of extracts of human Sup T1 cells transfected with the vectors shown. The markers are in kilodaltons, gp 160 and gp 120 mark the appropriate proteins, and 517 + tat is the positive control (HIV LTR driving env in the presence of tat).

Figure 3 depicts the protocol for testing T-cell killing induced in mice injected with syngeneic tumor cells expressing HIV env (the vector is pAF/env-SV-Neo).

Figure 4 graphically depicts the results of the experimental protocol in Figure 3. The specific killing is seen in the top graph with BC10MEenV-29 killing vs. B/C10ME resistance to killing.

Figure 5 diagrammatically illustrates the number of cells surviving after phleomycin selection upon transfection of cells with a plasmid which expresses the phlemoycin resistance gene (PRG) directly from a promoter (right) and with another which expresses PRG with a coding sequence interposed between it and the promoter.

Figure 6 depicts four plasmids designed to express retroviral proteins in mammalian cells. pSVgp and pRSVenv are cotransfectd with a selectable marker, while pSVgp-DHFR and pRSVenv-phleo are the equivalent plasmids with the selectable marker placed downstream of the viral protein-coding regions.

Figure 7 depicts three sites of fusion of HIV env and MLV env after site-directed mutagenesis of both coding sequences to create new compatible restriction enzyme sites. The N terminal sequences are at the left in both cases. The numbering is according to nucleotide numbers. ST, SR, SE are the starts of tat, rev and env while TT, TR, and TE are the corresponding termination sites.

Figure 8 depicts the substitution of U3 in a 5' LTR by a heterologous promoter/enhancer which can be fused to either the Sac I, Bssh II or other site in the region.

Figure 9 illustrates a representative method for crossing transgenic mice expressing viral protein or vector RNA.

### Detailed Description of the Invention

### I. Immunostimulation

The ability to recognize and defend against foreign pathogens is central to the function of the immune system. This system, through immune recognition, must be capable of distinguishing "self" from "nonself" (foreign), which is essential to ensure that defensive mechanisms are directed toward invading entities rather than against host tissues. The fundamental features of the immune system are the presence of highly polymorphic cell surface recognition structures (receptors) and effector mechanisms (antibodies and cytolytic cells) for the destruction of invading pathogens.

Cytolytic T lymphocytes (CTL) are induced normally by the display of processed pathogen-specific peptides in conjunction with the MHC class I or class II cell surface proteins. Within one embodiment of the present invention, presentation of immunogenic viral determinants in the context of appropriate MHC molecules efficiently induces optimal CTL responses without exposing the patient to the pathogen. This vector approach to immunostimulation provides a more effective means of inducing protective and therapeutic CTL responses, because the type of immunity induced by the vector more closely resembles that induced by exposure to natural infection. Based on current knowledge of several viral systems, it is unlikely that exogenously supplied, nonreplicating viral antigens, such as peptides and purified recombinant proteins, will provide sufficient stimulus to induce optimal class I-restricted CTL responses. Alternatively, vector-delivered expression of selected viral proteins within target cells as described within the present invention provides such a stimulus.

By way of example, in the case of HIV-1 infections, patients develop antibodies specific for a variety of viral envelope-region determinants, some of which are capable of in vitro virus neutralization. Nevertheless, disease progression continues and the patients eventually succumb to the disease. Low-level CTL responses against infected patients' cells (Plata et al., Nature 328:348-351, 1987) and against target cells infected with recombinant vaccinia vectors expressing HIV gag, pol, or env (Walker et al., Nature 328:345-348, 1987; Walker et al., Science 240:64-66, 1988) have been detected in some HIV-1 seropositive patients. In addition, it has recently been shown that murine as well as human CTL can be induced by autologous stimulator cells expressing HIV gp 120 via transfection (Langlade-Demoyan et al., J. Immunol. 141:1949, 1988). Improved CTL induction could be therapautically advantageous to injected patients and provide effective preventive therapy to individuals under noninfectious conditions. HIV infection itself may not be producing adequate CTL response because other elements associated with HIV infection may prevent proper immune stimulation. In addition, it may be that stimulation of T-cells by infected cells is an interaction that leads to infection of the stimulated T-cells.

Example 1 describes procedures for constructing plasmids capable of generating retroviral vectors in packaging cells, which then lead to expression of HIV viral antigens.

### EXAMPLE 1

### Vectors Expressing HIV Antigens

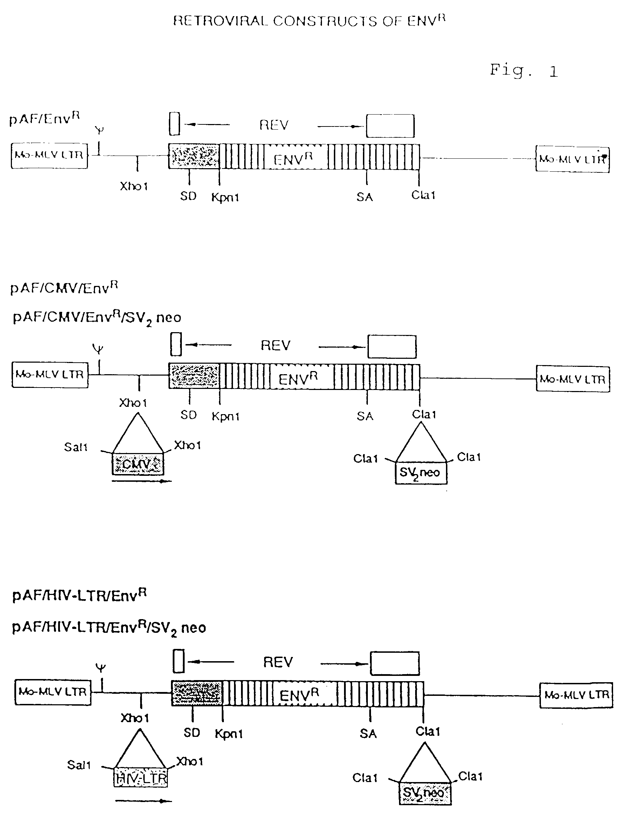

### A. Env Expression Vector (See Figure 1):

A 2.7 kb Kpn-Xho I DNA fragment was isolated from the HIV proviral clone BH10-R3 (for sequence, see Ratner et al., Nature 313:277, 1985) and a ≈400 bp Sal-Kpn I DNA fragment from IIIexE7deltaenv (a Bal31 deletion to nt. 5496) was ligated into the Sal I site in the plasmid SK⁺. From this clone, a 3.1 kb env DNA fragment (Xho I-Cla I) which also encodes rev, essential for env expression, was purified and ligated into a retroviral vector called pAFVXM (see Kriegler et al., Cell 38:483, 1984). This vector was modified in that the Bgl II site was changed by linker insertion to a Xho I site to facilitate cloning of the HIV env coding DNA fragment.

A dominant selectable marker gene comprised of a SV40 early promoter driving expression of neomycin phosphotransferase gene was inserted into the vector at the Cla I site to facilitate isolation of infected and transfected cell lines.

The Xho I site upstream from the ENV gene in the vector provides a convenient site to insert additional promoters into the vector construct as the RSV promoter, SV40 early or late promoter, the CMV immediate early (IE) promoter, human beta-bactin promoter, and Moloney murine MLV SL3-3 promoter.

One such promoter, the CMV Immediate Early gene promoter, a 673 bp DNA fragment Hinc II to Eag I, results in a tenfold increase in ENV expression in a human T-cell line called Sup TI when compared to the parental construct pAF ENV^{r} SV₂ Neo.

### B. Gag Expression Vector:

A 2.5 kb Sac I-Eco RV DNA fragment was isolated from pBH10-R3 (see Ratner et al., op. cit.) and ligated into the Sac I-Sal I site of pUC31. pUC31 is derived from pUC19 with additional Xho I, Bgl II, Bsst II and Nco I sites inserted between the Eco R1 and Kpn I sites of the poly linker. However, this construct contained the major splice donor (SD) site from HIV and thus could be problematic in virus generation. The SD site was removed by subcloning a 70 bp Rsa I-Cla I fragment with a 2.1 kb Cla I-Bam H1 DNA fragment into the Hinc II-BamH1 site of SK⁺. The Bam H1 site was converted into a Cla I site by linker insertion. This construct was designated SK⁺ gag protease SD delta.

The 2.5 kb Xho I-Cla I DNA fragment from SK⁺ gag protease SD delta was inserted into the Xho I/Cla I sites of the vector pAFVXM just as described above.

These plasmids, when placed in a suitable packaging cell, expressed a retroviral vector construct which contains a packaging signal. The packaging signal directed packaging of the vector construct into a capsid and envelope along with all further proteins required for viable retroviral particles. The capsid, envelope, and other proteins are preferably produced from one or more plasmids containing suitable genomes placed in the packaging cell. Such genomes may be proviral constructs, wich in a simple case may merely have the packaging signal detected. As a result, only the vector will be packaged. Suitable packaging or packaging cell lines, and the genome necessary for accomplishing such packaging, are described in Miller et al. (Mol. Cell. Bio. 6:2895, 1986).

As described by Miller et al., it is preferable that further changes be made to the proviral construct other than simple deletion of the packaging signal in order to reduce the chances of recombination events occurring within the packaging cell line, which may result in production of viral particles which are not replication defective.

It will be understood that Example 1 is merely illustrative of a procedure for generating an HIV envelope glycoprotein (gp) or other viral antigen. It is also possible to provide a proviral vector construct which expresses a modified HIV envelope gp on the target cells which will likewise stimulate an immune response, but with less T-cell cytopathic effects. Envelope glycoproteins can be suitably modified using techniques well known in the art, for instance through use of the disclosure of articles such as Kowalski et al. (Science 237:1351, 1987).

Thus, a proviral construct may be constructed by the above technique which generates retroviral constructs expressing such a suitably modified gp. This construct is then placed into a packaging cell as described above. The resulting recombinant retroviruses produced from the packaging cell lines may be used in vitro and in vivo to stimulate an immune response through the infection of susceptible target cells. It will be appreciated that other proteins expressed from the HIV genome, such as gag, pol, vif, nef, etc., may also elicit beneficial cellular responses in HIV-infected individuals. Proviral vectors such as those described below are designed to express such proteins so as to encourage a clinically beneficial immune response. It may be necessary for certain vectors to include rev coding sequences as well as a rev responsive element (Rosen et al., Proc. Natl. Acad. Sci. 85:2071, 1988).

The following example demonstrates the ability of this type of treatment to elicit CTL responses in mice.

### EXAMPLE 2

### Immune Response to Retroviral Vector-Encoded Antigens

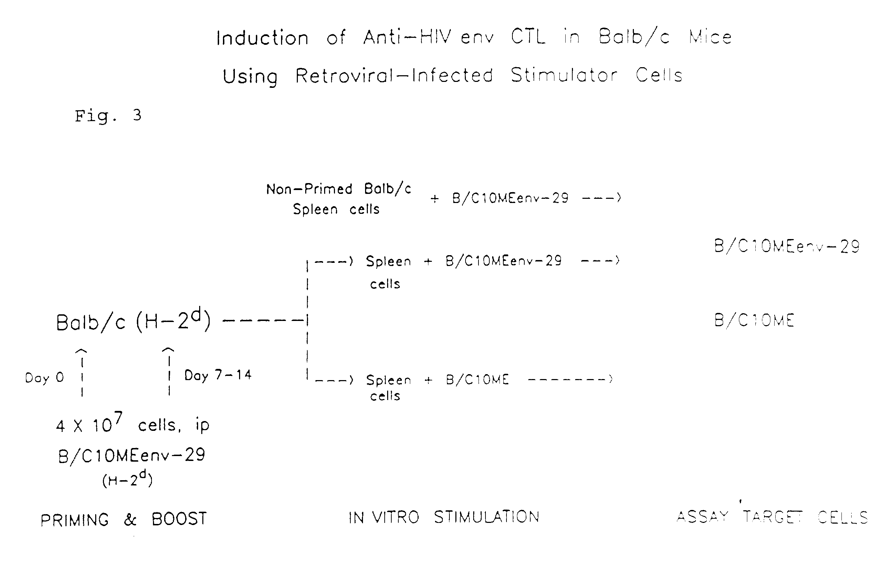

A murine tumor cell line (B/C10ME) (H-2^{d}) was infected with the pAF env^{r}SV40 Neo vector construct coding for HIV env. One cloned HIV-env expressing cell line (B/C10ME-29) was then utilized to stimulate HIV-env-specific CTL in syngeneic (i.e., MHC identical) Balb/c (H-2^{d}) mice. Mice were immunized by intraperitoneal injection with B/C10ME-29 cells (4 x 10⁷ cells) and boosted on day 7-14. Responder spleen cell suspensions were prepared from these immunized mice and the cells cultured in vitro for 4 days in the presence of either B/C10ME-29 or B/C10ME mitomycin-C-treated cells at a stimulator:responder cell ratio of 1:50 (Figure 3). The effector cells were harvested from these cultures, counted, and mixed with radiolabeled (⁵¹Cr) target cells (i.e., B/C10MEenv-29 or B/C10ME) at various effector:target (E:T) cell ratios in a standard 4-5 hr ⁵¹Cr-release assay. Following incubation, the microtiter plates were centrifuged, 100 µl of culture supernant was removed, the amount of radiolabel released from lysed cells quantitated in a Beckman gamma spectrometer. Target cell lysis was calculated as: **%** Target Lysis - Exp CPM - SR CPM/MR CPM - SR CPM x 100, where experimental counts per minute (Exp CPM) represents effectors plus targets; spontaneous release (SR) CPM represents targets alone; and maximum release (MR) CPM represents targets in the presence of 1 M HCL.

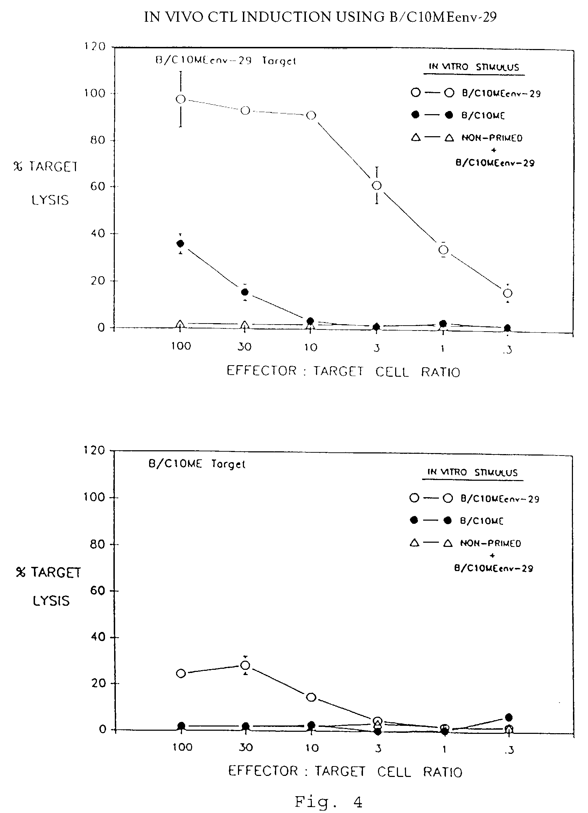

The results (Figure 4) illustrate that CTL effectors were induced which specifically lysed HIV-env-expressing target cells (B/C10MEenv-29) significantly more efficiently than B/C10ME targets. Primed spleen cells restimulated in vitro with non-HIV-env-expressing control cells (B/Cl0ME) did not show significant CTL activity on either B/ClOMEenv-29 or B/C10Me targets, particularly at lower E:T cell ratios. Spleen cells obtained from naive nonimmunized Balb/c mice which were restimulated in vitro with B/C10MEenv-29 did not generate CTLS, thus suggesting the importance of the in vivo priming and boosting event. This experiment has been repeated and similar results obtained.

In another experiment, effector cells obtained from Balb/c mice immunized, boosted and restimulated in vitro with a different H-2^{d} HIV-env-expressing tumor cell clone (L33-41) infected with the same pAF env^{r} SV40 Neo (HIV-env) vector construct were capable of lysing B/C10MEenv-29 target cells. This provides additional support that the CTL generated in these mice are specifically recognizing an expressed form of HIV-env rather than simply a unique tumor cell antigen on these cells. This result also suggests that the vector-delivered antigen is presented in a similar manner by the two tumor cell lines.

Implementation of this immunostimulant application in humans requires that (1) the gene coding for the antigen of interest be delivered to cells, (2) the antigen be expressed in appropriate cells, and (3) MHC restriction requirements, i.e., class I and class II antigen interaction, are satisfied. Preparations of vector are made by growing the producer cells in normal medium, washing the cells with PBS plus human serum albumin (HSA) at 10 mg/ml, then growing the cells for 8-16 hours in PBS plus HSA. Titers obtained are typically 10⁴ to 10⁶/ml depending on the vector, packaging line or particular producer line clone. The vector supernatants are filtered to remove cells and are concentrated up to 100-fold by filtration through 100,000 or 300,000 MW pass Amicon filters (Wolff et al., Proc. Natl. Acad. Sci. 84:3344, 1987). This lets globular proteins of 100,000 or 300,000 MW pass but retains 99% of the viral vector as infectious particles. The stocks can be frozen for storage since they lose about 50% of the infectious units on freezing and thawing. The most direct delivery involves administration of the appropriate gene-carrying vector into the individual and reliance upon the ability of the vector to efficiently target to the appropriate cells, which can then initiate stimulation of the immune response. The dose would be 10⁵ - 10⁶ infectious units/kg body weight. However, a more practical approach may involve the extracorporeal treatment of patient peripheral blood lymphocytes (PBL), fibroblasts or other cells obtained from each individual with the vector. PBL could be maintained in culture through the use of mitogens (phytohemagglutinin) or lymphokines (e.g., IL-2). This type of approach allows for directed vector infection, monitoring of expression and expansion of the antigen presenting cell population prior to injection, and return of vector-expressing cells to the respective patient. Other types of cells could also be explanted, vector introduced, and the cells returned to the patient. Only a moderate number of infected cells (10⁵-10⁶/kg body weight) is necessary to elicit strong immune responses.

A different form of administration is the implantation of producer lines making retroviral vector particles. These may be immunologically unmatched classical producers cell lines or the patients' own cells, which have been explanted, treated and returned (see Alternative Viral Vector Packaging Techniques, below). Both types of implants (10⁵ - 10⁶/kg body weight) would have a limited life span in the patient, but would lead to the retroviral vector infecting large numbers (10⁷ - 10¹⁰) of cells in their vicinity in the body.

In any case, the success of the treatment can be assayed by removing a small amount of blood and measuring the CTL response using as targets the individual's own cells infected with vector leading to env expression.

When it is desired to stimulate an MHC class I or class II restricted immune response to pathogens, including pathogenic viruses other than HIV, suitable forms of envelope or other antigens associated with such retroviruses which will stimulate an immune response can be ascertained by one skilled in the art. In general, suitable forms of antigens associated with pathogenic agents can readily be selected which will stimulate an immune response to those pathogenic agents.

Ex corpore treatment of patients' cells would aim, for example, for uninfected potentially susceptible T-cells or monocytes. One preferred method of targeting the susceptible cell is with vectors which carry HIV env or hybrid env (see section headed "Cell-line Specific Retroviruses", below) to direct absorption of vector particles to CD4* cells. Normal adults have about 5 x 10⁹ T4 cells in their total blood and about the same number of monocytes.

A preferred method of administration would be leukophoresis, wherein about 20% of an individual's PBLs can be removed at any one time and manipulated in vitro. Thus, about 2 x 10⁹ cells would be treated and replaced. Since the current maximum titres are around 10⁶/ml, this would require 2 to 20 liters of starting viral supernatant. Repeat treatments would be performed.

Another route which may be used is the aspiration of bone marrow, infection with retroviral vector and return of this infected marrow (Gruber et al., Science 230: 1057, 1985) to the patient. The marrow replication will amplify the vector expression through cell replication.

In any case, the efficacy of the treatment can be assayed by measuring the usual indicators of disease progression, including antibody level, viral antigen production, infectious HIV levels or levels of non-specific infections.

### Packaging Cell Selection

Basically, at least five factors may be postulated as causes for low recombinant virus titres:
1. the limited availability of viral packaging proteins;
2. the limited availability of retroviral vector RNA genomes;
3. the limited availability of cell membrane for budding of the recombinant retroviruses;
4. the limited intrinsic packaging efficiency of the retroviral vector genome; and
5. the density of the receptor specific for the envelope of a given retrovirus.

As noted above, the limited availability of viral packaging proteins is the initial limiting factor in recombinant retrovirus production from packaging cells. When the level of packaging protein in the packaging cells is increased, titre increases to about 10⁵ infectious units/milliliter, following which increasing packaging protein level has no further effect on titres. However, titres can be further augmented by also increasing the level of retroviral vector genome available for packaging. Thus, as described herein, it is advantageous to select producer cells that manufacture the maximum levels of packaging proteins and retroviral vector genomes. It has been discovered that the methods of identifying, and thus selecting, packaging cells and producer cells, described earlier under the section entitled "Background of the Invention," tend to lead to selection of many producer cells which produce low titres for the reasons described below.

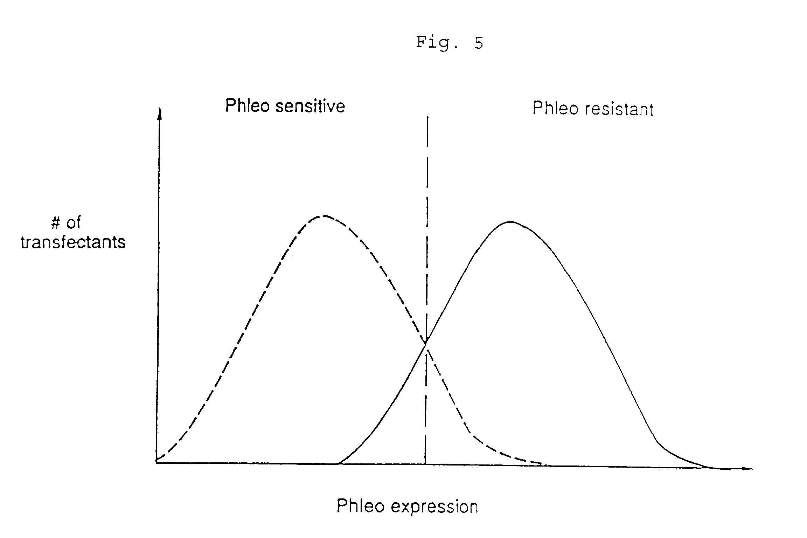

In obtaining packaging cell lines, advantage may be taken of the previously disadvantageous fact that the protein expression level of a gene downstream from the 5' LTR or other promoter, and spaced therefrom by an intervening gene, is substantially less than if the intervening gene were absent. Thus, the selectable gene is placed downstream from a gene of the packaging genome or the gene of interest carried by the vector construct, but is still transcribed under the control of the viral 5' LTR or other promoter without any splice donor or splice acceptor sites. This accomplishes two things. First, since the packaging genes or genes of interest are now upstream with no intervening gene between themselves and the promoter, their corresponding proteins (packaging protein or protein of interest) will be expressed at a higher level (5- to 20-fold) than the selectable protein. Second, the selectable protein will be expressed on average at a lower level, with the distribution of level of expression shifting toward lower levels. In the case of the phleo^{r} protein, this shift in distribution is illustrated by the broken curve indicated in Figure 5. However, the selection level for resistance to phleomycin remains the same, so that only the top-end expressing cells survive. The levels of the packaging protein or of the protein of interest will still be proportional, only in this case, a higher level of selectable protein corresponds to a much higher level of packaging protein or protein of interest.

Preferably, the foregoing procedure is performed using a plasmid carrying one of the proviral gag/pol or env packaging genes, along with a first selectable gene. These cells are then screened for the cells producing the highest levels of protein by reaction with an antibody against env (or possibly gag/pol), a second fluorescent antibody, and then sorted on a fluorescence-activated cell sorter (FACS). Alternatively, other tests for protein level may be used. Subsequently, the procedure and screening are repeated using those selected cells, and the other of the gag/pol or env packaging genes. In this step, a second selectable gene (different from the first) would be required downstream from the packaging gene and the cells producing the largest amount of the second viral protein selected. The procedure and screening are then repeated using the surviving cells, with a plasmid carrying the proviral vector construct bearing the gene of interest and a third selectable gene, different from the first or second selectable gene. As a result of this procedure, cells producing high titres of the desired recombinant retrovirus will be selected, and these can be cultured as required to supply recombinant retrovirus. In addition, gag and pol can be independently introduced and selected for.

Example 3 describes the construction of gag/pol and env plasmids designed to use these procedures.

### EXAMPLE 3

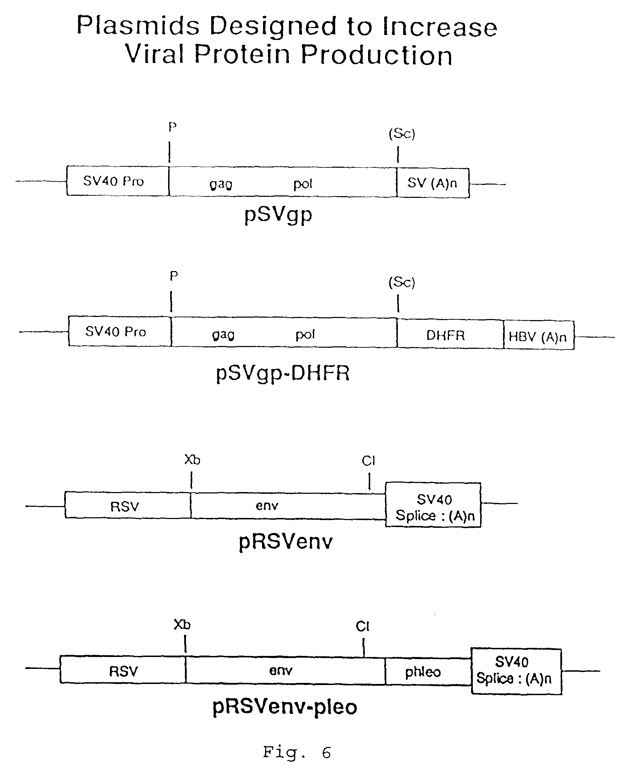

### Plasmids Designed to Make High Levels of Packaging Proteins (Figure 6)

1. The 2.7 kb Xba I fragment from pPAM (Miller et al., Mol. Cell. Biol. 5:431, 1985), which contains the amphotropic env segment, was cloned in pUC18 at the Xba I site, then removed with Hind III and Sma I. This fragment was cloned into the vector pRSV neo (Gorman et al., Mol. Cell. Biol. 2:1044, 1982; Southern et al., J. Mol. Appl. Genet. 1:327, 1982) cut with Hind III and Pvu II, to give pRSV env. A 0.7 kb Bam H1 to BstE II fragment from the plasmid pUT507 (Mulsant et al., Somat. Cell. Mol. Genet. 14:243, 1988) with the BstE II end filled in carries the phleo resistance coding sequence. The 4.2 kb Bam H1 to Xho I fragment, the contiguous 1.6 kb Xho I to Xba I (Xba I filled in) from RSVenv, and the phleo fragment were ligated to give pRSVenv-phleo.
2. A fragment from the Pst I site at nucleotide 563 of MLY (RNA Tumor Viruses, Vol. II, 1985, Cold Spring Harbor) to the Sca I site at 5870 was derived from pMLV-K (Miller et al., 1985, op. cit.) and cloned in the Pst I to Bam H1 (Bam H1 filled-in) fragment from p4aA8 (Jolly et al., Proc. Natl. Acad. Sci. USA 80:477, 1983) that has the SV40 promoter, the pBR322 ampicillin resistance and origin of replication and the SV40 poly A site. This gives pSVgp. pSVgpDHFR was made using the following fragments: the 3.6 kb Hind III to Sal I fragment from pSVgp containing the SV40 promoter plus MLY gag and some pol sequences; the 2.1 kb Sal I to Sca I fragment from pMLV-K with the rest of the pol gene, the 3.2 kb Xba I (Xba I filled-in) to Pst I fragment from pF400 with the DHFR gene plus poly A site, pBR322 origin and half the ampicillin resistance gene; the 0.7 kb Pst I to Hind III fragment from pBR322 with the other half of the ampicillin resistance gene. This gives pSVgp-DHFR. All these constructs are shown in Figure 6. These plasmids can be transfected into 3T3 cells or other cells and high levels of gag, pol or env obtained.

An additional method for accomplishing selection is to use a gene selection in one round and its antisense in a subsequent round. For example, gag/pol may be introduced into an HPRT-deficient cell with the HPRT gene and selected for the presence of this gene using that media which requires HPRT for the salvage of purines. In the next round, the antisense to HPRT could be delivered downstream to env and the cell selected in 6 thioguanine for the HPRT-deficient phenotype. Large amounts of antisense HPRT would be required in order to inactivate the HPRT gene transcripts, assuming no reversion occurred.

### Envelope Substitutions

The ability to express gag pol and env functions separately allows for manipulation of these functions independently. A cell line that expresses ample amounts of gag pol can be used, for example, to address questions of titre with regard to env. One factor resulting in low titres is the density of appropriate receptor molecules on the target cell or tissue. Given that env expression is from a separate unit, a variety of envelope genes (requiring different receptor proteins), such as xenotropic, polytropic, or amphotropic envs from a variety of sources, can be tested for highest titres on a specific target tissue. Furthermore, envelopes from nonmurine retrovirus sources can be used for pseudotyping a vector. In addition, hybrid envelopes (as described below) can be used in this system as well, to tailor the tropism (and effectively increase titres) of a retroviral vector. Conversely, a cell line that expresses ample amounts of a given envelope gene can be employed to address questions of titre with regard to gag and pol.

### Alternative Viral Vector Packaging Techniques

Two additional alternative systems can be used to produce recombinant retroviruses carrying the vector construct. Each of these systems takes advantage of the fact that the insect virus, baculovirus, and the mammalian viruses, vaccinia and adenovirus, have been adapted recently to make large amounts of any given protein for which the gene has been cloned. For example, see Smith et al. (Mol. Cell. Biol. 3:12, 1983); Piccini et al. (Meth. Enzymology, 153:545, 1987); and Mansour et al. (Proc. Natl. Acad. Sci. USA 82:1359, 1985).

These viral vectors can be used to produce proteins in tissue culture cells by insertion of appropriate genes into the viral vector and, hence, could be adapted to make retroviral vector particles.

Adenovirus vectors are derived from nuclear replicating viruses and can be defective. Genes can be inserted into vectors and used to express proteins in mammalian cells either by in vitro construction (Ballay et al., EMBO J. 4:3861, 1985) or by recombination in cells (Thummel et al., J. Mol. Appl. Genetics 1:435, 1982).

One preferred method is to construct plasmids using the adenovirus Major Late Promoter (MLP) driving: (1) gag/pol, (2) env, (3) a modified viral vector construct. A modified viral vector construct is possible because the U3 region of the 5' LTR, which contains the viral vector promoter, can be replaced by other promoter sequences (see, for example, Hartman, Nucl. Acids Res. 16:9345, 1988). This portion will be replaced after one round of reverse transcriptase by the U3 from the 3' LTR.

These plasmids can then be used to make adenovirus genomes in vitro (Ballay et al., op. cit.), and these transfected in 293 cells (a human cell line making adenovirus E1A protein), for which the adenoviral vectors are defective, to yield pure stocks of gag, pol, env and retroviral vector carried separately in defective adenovirus vectors. since the titres of such vectors are typically 10⁷-10¹¹/ml, these stocks can be used to infect tissue culture cells simultaneously at high multiplicity. The cells will then be programmed to produce retroviral proteins and retroviral vector genomes at high levels. since the adenovirus vectors are defective, no large amounts of direct cell lysis will occur and retroviral vectors can be harvested from the cell supernatants.

In an alternative system (which is more truly extracellular), the following components are used:
1. gag/pol and env proteins made in the baculovirus system in a similar manner as described in Smith et al. (supra) (or in other protein production systems, such as yeast or E. coli);
2. viral vector RNA made in the known T7 or SP6 or other in vitro RNA-generating system (see, for example, Flamant and Sorge, J. Virol. 62:1827, 1988);
3. tRNA made as in (2) or purified from yeast or mammalian tissue culture cells;
4. liposomes (with embedded env protein); and
5. cell extract or purified necessary components (when identified) (typically from mouse cells) to provide env processing, and any or other necessary cell-derived functions.

Within this procedure (1), (2) and (3) are mixed, and then env protein, cell extract and preliposome mix (lipid in a suitable solvent) added. It may, however, be necessary to earlier embed the env protein in the liposomes prior to adding the resulting liposome-embedded env to the mixture of (1), (2), and (3). The mix is treated (e.g., by sonication, temperature manipulation, or rotary dialysis) to allow encapsidation of the nascent viral particles with lipid plus embedded env protein in a manner similar to that for liposome encapsidation of pharmaceuticals, as described in Gould-Fogerite et al., Anal. Biochem. 148:15, 1985). This procedure allows the production of high titres of replication incompetent recombinant retroviruses without contamination with pathogenic retroviruses or replication-competent retroviruses.

### Cell Line-Specific Retroviruses "Hybrid Enveloppe"

The host cell range specificity of a retrovirus is determined in part by the env gene products. For example, Coffin, J. (RNA Tumor Viruses 2:25-27, Cold spring Harbor, 1985) notes that the extracellular component of the proteins from murine leukemia virus (MLV) and Rous sarcoma virus (RSV) are responsible for specific receptor binding. The cytoplasmic domain of envelope proteins, on the other hand, are understood to play a role in virion formation. While pseudotyping (i.e., the encapsidation of viral RNA from one species by viral proteins of another species) does occur at a low frequency, the envelope protein has some specificity for virion formation of a given retrovirus. By creating a hybrid env gene product (i.e., specifically, an env protein having cytoplasmic regions and exogenous binding regions which are not in the same protein molecule in nature) the host range specificity may be changed independently from the cytoplasmic function. Thus, recombinant retroviruses can be produced which will specifically bind to preselected target cells.

In order to make a hybrid protein in which the receptor binding component and the cytoplasmic component are from different retroviruses, a preferred location for recombination is within the membrane-spanning region of the cytoplasmic component. Example 4 describes the construction of a hybrid env gene which expresses a protein with the CD4 binding portion of the HIV envelope protein coupled to the cytoplasmic domain of the MLV envelope protein.

### EXAMPLE 4

### Hybrid HIV-MLV Envelopes

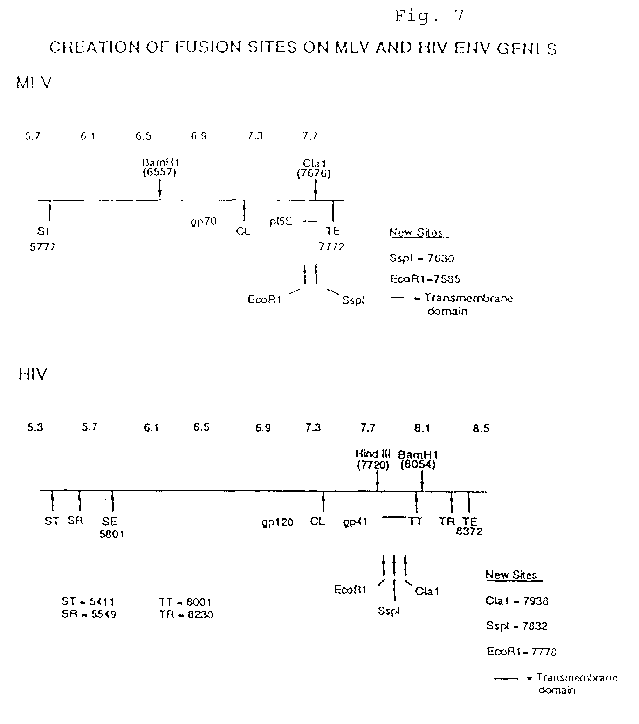

A hybrid envelope gene is prepared using in vitro mutagenesis (Kunkel, Proc. Natl. Acad. Sci. USA 82:488-492, 1985) to introduce a new restriction site at an appropriate point of junction. Alternatively, if the two envelope sequences are on the same plasmid, they can be joined directly at any desired point using in vitro mutagenesis. The end result in either case is a hybrid gene containing the 5' end of the HIV gp 160 and the 3' end of MLB p15E. The hybrid protein expressed by the resulting recombinant gene is illustrated in Figure 7 and contains the HIV gp120 (CD4 receptor binding protein), the extracellular portion of HIV gp 41 (the gp 120 binding and fusigenic regions), and the cytoplasmic portion of MLV pl5E, with the joint occurring at any of several points within the host membrane. A hybrid gene made by fusion at the new Eco R1 site shown in Figure 7 is expressed under control of the CMVIE gene in human Sup T1 cells and leads to syncytia, as is the case with the authentic HIV env gene. Thus the hybrid is correctly transported to the cell surface and displayed there.

While Example 4 illustrates one hybrid protein produced from two different retroviruses, the possibilities are not limited to retroviruses or even simply other viruses. For example, the beta-receptor portion of human interleukin-2 may be combined with the envelope protein of MLV. In this case, a recombination would preferably be located in the gp 70 portion of the MLV env gene, leaving an intact p15E protein. Furthermore, the foregoing technique may be used to create a recombinant retrovirus with an envelope protein which recognizes antibody Fc segments. Monoclonal antibodies which recognize only preselected target cells only could then be bound to such a recombinant retrovirus exhibiting such envelope proteins so that the retrovirus would bind to and infect only those preselected target cells.

### Site-Specific Integration

Targeting a retroviral vector to a predetermined locus on a chromosome increases the benefits of gene-delivery systems. A measure of safety is gained by direct integration to a "safe" spot on a chromosome, i.e., one that is proven to have no deleterious effects from the insertion of a vector. Another potential benefit is the ability to direct a gene to an "open" region of a chromosome, where its expression would be maximized. Two techniques for integrating retroviruses at specific sites are described below.

### (i) Homologous Recombination

One technique for integrating an exogenous gene of a vector construct of a recombinant retrovirus into a specific site in a target cell's DNA employs homologous recombination. Plasmids containing sequences of DNA of greater than about 300 bp that are homologous to genomic sequences have been shown to interact (either by replacement or insertion) with those genomic sequences at a rate that is greater than 10³-fold over a specific interaction in the absence of such homology (see Thomas and Capecchi, Cell 51:503-12, 1987; and Doetscheman et al., Nature 330:576-78, 1987). It has been shown that an insertion event, or alternatively, a replacement event, may be driven by the specific design of the vector.

In order to employ homologous recombination in site-specific retroviral integration, a vector construct should be modified such that (a) homologous sequences (to the target cell's genome) are incorporated into the construct at an appropriate location; and (b) the normal mechanism of integration does not interfere with the targeting occurring due to homologous sequences. A preferred approach in this regard is to add homologous sequences (greater than about 300 bp) in the 3' LTR, downstream from the U3 inverted repeat. In this approach, the construct is initially made with a region of homology inserted in the 3' LTR at the Nhe 1 site in U3. Reverse transcription in the host cell will result in a duplication of the region of homology in the 5' LTR within 31 bp of the end of the inverted repeat (IR). Integration into the host genome will occur in the presence or absence of the normal integration mechanism. The gene in the vector may be expressed, whether from the LTR or from an internal promoter. This approach has the effect of placing a region of homology near a potential free end of the double-stranded retrovirus vector genome. Free ends are known to increase the frequency of homologous recombination by a factor of approximately 10. In this approach, it may be necessary to defeat the normal mechanism of integration, or to at least modify it to slow down the process, allowing time for homologous DNAs to line up. Whether this latter modification is required in a particular case can be readily ascertained by one skilled in the art.

### (ii) Integrase Modification

Another technique for integrating a vector construct into specific, preselected sites of a target cell's genome involves integrase modification.

The retrovirus pol gene product is generally processed into four parts: (i) a protease which processes the viral gag and pol products; (ii) the reverse transcriptase; and (iii) RNase H, which degrades RNA of an RNA/DNA duplex; and (iv) the endonuclease or "integrase."

The general integrase structure has been analyzed by Johnson et al. (Proc. Natl. Acad. Sci. USA 83:7648-7652, 1986). It has been proposed that this protein has a zinc-binding finger with which it interacts with the host DNA before integrating the retroviral sequences.

In other proteins, such "fingers" allow the protein to bind to DNA at particular sequences. One illustrative example is the steroid receptors. In this case, one can make the estrogen receptor, responding to estrogens, have the effect of a glucocorticoid receptor, responding to glucocorticoids, simply by substituting the glucocorticoid receptor "finger" (i.e., DNA binding segment) in place of the estrogen receptor finger segment in the estrogen receptor gene. In this example, the position in the genome to which the proteins are targeted has been changed. Such directing sequences can also be substituted into the integrase gene in place of the present zinc finger. For instance, the segment coding for the DNA binding region of the human estrogen receptor gene may be substituted in place of the DNA binding region of the integrase in a packaging genome. Initially, specific integration would be tested by means of an in vitro integration system (Brown et al., Cell 29:347-356, 1987). To confirm that the specificity would be seen in vivo, this packaging genome is used to make infectious vector particles, and infection of and integration into estrogen-sensitive and estrogen-nonsensitive cells compared in culture.

Through use of this technique, incoming viral vectors may be directed to integrate into preselected sites on the target cell's genome, dictated by the genome-binding properties of site-specific DNA-binding protein segments spliced into the integrase genome. It will be understood by those skilled in the art that the integration site must, in fact, be receptive to the fingers of the modified integrase. For example, most cells are sensitive to glucocorticoids and hence their chromatin has sites for glucocorticoid receptors. Thus, for most cells, a modified integrase having a glucocorticoid receptor finger would be suitable to integrate the proviral vector construct at those glucocorticoid receptor-binding sites.

### Production of Recombinant Retroviral Vectors in Transgenic Animals

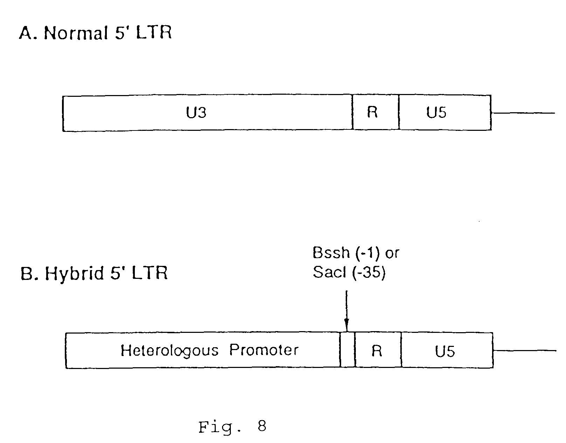

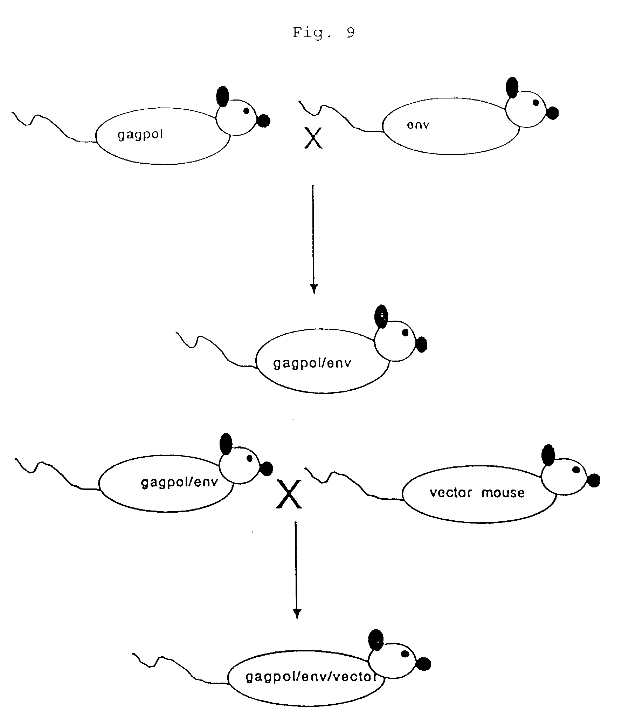

Two problems previously described with helper line generation of retroviral vectors are: (a) difficulty in generating large quantities of vectors; and (b) the current need to use permanent instead of primary cells to make vectors. These problems can be overcome with producer or packaging lines that are generated in transgenic animals. These animals would carry the packaging genomes and retroviral vector genomes. Current technology does not allow the generation of packaging cell lines and desired vector producing lines in primary cells due to their limited life span. The current technology is such that extensive characterization is necessary, which eliminates the use of primary cells because of senescence. However, individual lines of transgenic animals can be generated by the methods provided herein which produce the packaging functions, such as gag, pol or env. These lines of animals are then characterized for expression in either the whole animal or targeted tissue through the selective use of housekeeping or tissue-specific promoters to transcribe the packaging functions. The vector to be delivered is also inserted into a line of transgenic animals with a tissue-specific or housekeeping promoter. As discussed above, the vector can be driven off such a promoter substituting for the U3 region of the 5' LTR (Figure 8). This transgene could be inducible or ubiquitous in its expression. This vector, however, is not packaged. These lines of animals are then mated to the gag/pol/env animal and subsequent progeny produce packaged vector. The progeny, which are essentially identical, are characterized and offer an unlimited source of primary producing cells. Alternatively, primary cells making gag/pol and env and derived from transgenic animals can be infected or transfected in bulk with retrovirus vectors to make a primary cell producer line. Many different transgenic animals or insects could produce these vectors, such as mice, rats, chickens, swine, rabbits, cows, sheep, fish and flies. The vector and packaging genomes would be tailored for species infection specificity and tissue-specific expression through the use of tissue-specific promoters and different envelope proteins. An example of such a procedure is illustrated in Figure 9.

Although the following examples of transgenic production of primary packaging lines are described only for mice, these procedures could be extended to other species by those skilled in the art. Given the homology to MLV sequences in mice genome, the final preferred animals would not be mice.

### Example 5

### Production of Gag-Pol Proteins Using Housekeeping Promoters for Ubiquitous Expression in Transgenic Animals

An example of a well-characterized housekeeping promoter is the HPRT promoter. HPRT is a purine salvage enzyme which expresses in all tissues. (See Patel et al., Mol. Cell Biol. 6:4 -403, 1986 and Melton et al., Proc. Natl. Acad. Sci, 81:2147-2151, 1984). This promoter would be inserted in front of various gag/pol fragments (e.g., Bal I/Sca I; Aat II/Sca I; Pst I/Sca I of MoMLV; see RNA Tumor Viruses 2, 1985, Cold Spring Harbor Laboratory, 1985) that are cloned in Bluescript plasmids (Strategene, Inc.) using recombinant DNA techniques (see Maniatis et al., Molecular Cloning: A Laboratory Manual, Cold Spring Harbor, 1982). The resulting plasmids are purified (Maniatis et al., op. cit.) and the relevant genetic information isolated using Geneclean (Bio 101) or electroelution (see Hogan et al.(Eds:) Manipulating the Mouse Embryo: A Laboratory Manual, Cold Spring Harbor, 1986).

These fully characterized DNAs Would be microinjected in the pronucleus of fertilized mouse ova at a concentration of 2 µg/ml. Live born mice would be screened by tail blot analyses (see Hogan et al., op. cit.). Transgenic-positive animals would be characterized for expression levels of gag-pol proteins by immunoprecipitation of radiolabeled primary cells, such as fibroblasts (see Harlow et al., eds. Antibodies: A Laboratory Manual, Cold Spring Harbor, 1988). Animals would then be bred to homozygosity for establishment of animal lines that produce characterized levels of gag-pol.

### Example 6

### Production of env Proteins/Hybrid Envelope Proteins Using Housekeeping Promoters for Ubiquitous Expression in Transgenic Animals

This example utilizes the HPRT promoter for expression of either envelope or hybrid envelope proteins. The envelope proteins could be from any retrovirus that is capable of complementing the relevant gag-pol, in this case that of MLV. Examples are ecotropic MLV, amphotropic MLV, xenotropic MLV, polytropic MLV, or hybrid envelopes. As above, the envelope gene would be cloned behind the HPRT promoter using recombinant DNA techniques (see Maniatis et al., op. cit.). The resulting "minigene" would be isolated (see Hogan et al., op. cit.), and expression of envelope protein would be determined (Harlow et al., op. cit.). The transgenic envelope animals would be bred to homozygosity to establish a well-characterized envelope animal.

### Example 7

### Production of gag-pol-env Animals Usinq Housekeeping Promoters for Ubiquitous Expression in Transgenic Animals

This would use the well-characterized gag-pol animals, as well as the animals for the establishment of a permanent gag-pol/envelope animal line. This would involve breeding to homozygosity and the establishment of a well-characterized line. These lines would then be used to establish primary mouse embryo lines that could be used for packaging vectors in tissue culture. Furthermore, animals containing the retroviral vector could be bred into this line.

### Example 8

### Production of Tissue-Specific Expression of gag-pol-env or Hybrid Envelope in Transgenic Animals

The example given here is to direct tissue expression of the gagpol, envelope, or- hybrid envelope to specific tissues, such as T-cells. This involves the use of CD2 sequences (see Lang et al., EMBO J. 7:1675-1682, 1988) that give position and copy number independence. The 1.5 kb Bam H1/Hind III fragment from the CD2 gene would be inserted in front of gag-pol, envelope, or hybrid envelope fragments using recombinant DNA techniques. These genes would be inserted into fertilized mouse ova by microinjection. Transgenic animals would be characterized as before. Expression in T-cells would be established. Animals would be bred to homozygosity to establish well-characterized lines of transgenic animals. Gag-pol animals would be mated to envelope animals to establish gag-pol-env animals expressing only in T-cells. The T-cells of these animals would then be a source for T-cells capable of packaging retroviral vectors. Again, vector animals could be bred into these gag-pol-env animals to establish T-cells expressing the vector.

This technique allows the use of other tissue-specific promoters, such as milk-specific (whey), pancreatic (insulin or elastase), or neuronal (myelin basic protein) promoters. Through the use of promoters, such as milk-specific promoters, recombinant retroviruses may be isolated directly from the biological fluid of the progeny.

### Example 9

### Production of Either Housekeeping or Tissue-Specific Retroviral Vectors in Transgenic Animals

The insertion of retroviruses or retroviral vectors into the germ line of transgenic animals results in little or no expression. This effect, described by-Jaenisch (see Jahner et al., Nature 298:623-628, 1982), is attributed to methylation of 5' retroviral LTR sequences. This technique would overcome the methylation effect by substituting either a housekeeping or tissue-specific promoter to express the retroviral vector/retrovirus. The U3 region of the 5' LTR, which contains the enhancer elements, is replaced with regulatory sequences from housekeeping or tissue-specific promoters (see Figure 8). The 3' LTR is fully retained, as it contains sequences necessary for polyadenylation of the viral RNA and integration. As the result of unique properties of retroviral replication, the U3 region of the 5' LTR of the integrated provirus is generated by the U3 region of the 3' LTR of the infecting virus. Hence, the 3' is necessary, while the 5' U3 is dispensable. Substitution of the 5' LTR U3 sequences with promoters and insertion into the germ line of transgenic animals results in lines of animals capable of producing retroviral vector transcripts. These animals would then be mated to gag-pol-env animals to generate retroviral-producing animals (see Figure 9).

## Claims

1. A replication-defective recombinant retrovirus capable of infecting human cells carrying a vector construct capable of preventing, inhibiting, stabilizing or reversing infectious, cancerous or auto-immune diseases,
wherein the vector construct directs the expression of a disease antigen which is from a pathogenic agent or is a cancer antigen or modified form thereof in target cells infected with the retrovirus,
said antigen or modified form thereof being capable of stimulating a cell-mediated immune response within a human.

2. A recombinant retrovirus according to claim 1 wherein the vector construct directs the expression of a modified form of a disease antigen from a pathogenic agent in target cells infected with the retrovirus, said modified antigen being capable of stimulating an immune response within a human but having reduced pathogenicity relative to the pathogenic agent.

3. A recombinant retrovirus according to claim 1 or 2 wherein the expressed antigen elicits an HLA class I- or II- restricted immune response.

4. A recombinant retrovirus according to any one of claims 1 to 3 wherein the expressed antigen is an HIV protein selected from the group consisting of gp160, gp120 and gp41, or modified forms thereof.

5. A pharmaceutical composition comprising a recombinant retrovirus according to any one of claims 1 to 4 in combination with a physiologically acceptable carrier or diluent.

6. A recombinant retrovirus according to any one of claims 1 to 4 or a composition according to claim 5 for use as an active therapeutic substance in a method of treatment of the human body.

7. A method of producing a recombinant retrovirus as defined in any one of claims 1 to 4 comprising:
packaging a vector construct in a capsid and envelope such that said retrovirus is produced.

8. Ex vivo cells infected with a recombinant retrovirus according to any one of claims 1 to 4.

9. A eukaryotic cell capable of producing a replication-defective recombinant retrovirus according to any one of claims 1 to 4, said cell containing a retroviral genome for said recombinant retrovirus having a packaging signal and encoding a disease antigen which is from a pathogenic agent or is a cancer antigen or modified form thereof but lacking the coding sequence for one or more retroviral proteins and said cell also being capable of expressing viral proteins for production of said recombinant retrovirus.

10. Cells according to claim 8 or claim 9 for use in method of treatment of the human or animal body.

## Patentansprüche

1. Rekombinantes Retrovirus mit einem Replikationsdefekt mit der Fähigkeit, menschliche Zellen zu infizieren, das ein Vektorkonstrukt enthält, das infektiöse, canceröse oder Autoimmunerkrankungen verhüten, hemmen, stabilisieren oder umkehren kann, wobei das Vektorkonstrukt die Expression eines Krankheitsantigens, das von einem Krankheitserreger stammt oder ein Krebsantigen oder eine modifizierte Form davon ist, in Zielzellen, die mit dem Retrovirus infiziert sind, steuert, wobei das Antigen oder die modifizierte Form davon die zellvermittelte Immunantwort in einem Menschen stimulieren kann.

2. Rekombinantes Retrovirus nach Anspruch 1, wobei das Vektorkonstrukt die Expression einer modifizierten Form eines Krankheitsantigens von einem Krankheitserreger in Zielzellen steuert, die mit dem Retrovirus infiziert sind, wobei das modifizierte Antigen eine Immunantwort in einem Menschen stimulieren kann, aber eine verminderte Pathogenität, bezogen auf den Krankheitserreger, besitzt.

3. Rekombinantes Retrovirus nach Anspruch 1 oder 2, wobei das exprimierte Antigen eine auf die HLA-Klasse I oder II beschränkte Immunanwort hervorruft.

4. Rekombinantes Retrovirus nach einem der Ansprüche 1 bis 3, wobei das exprimierte Antigen ein HIV-Protein, ausgewählt aus der Gruppe, bestehend aus gp160, gp120 und gp41 oder eine modifizierte Form davon ist.

5. Pharmazeutisches Präparat, umfassend ein rekombinantes Retrovirus nach einem der Ansprüche 1 bis 4 in Kombination mit einem physiologisch verträglichen Träger oder Verdünnungsmittel.

6. Rekombinantes Retrovirus nach einem der Ansprüche 1 bis 4 oder ein Präparat nach Anspruch 5 zur Verwendung als aktive therapeutische Substanz in einem Verfahren zur Behandlung des menschlichen Körpers.

7. Verfahren zur Produktion eines rekombinanten Retrovirus wie in einem der Ansprüche 1 bis 4 definiert, wobei man ein Vektorkonstrukt in einem Capsid und einem Envelope so verpackt, daß das Retrovirus produziert wird.

8. Ex vivo-Zellen, infiziert mit einem rekombinanten Retrovirus nach einem der Ansprüche 1 bis 4.

9. Eukaryontische Zelle mit der Fähigkeit, ein rekombinantes Retrovirus mit einem Replikationsdefekt nach einem der Ansprüche 1 bis 4 zu produzieren, wobei die Zelle ein retrovirales Genom für das rekombinante Retrovirus enthält, das ein Verpakkungsignal enthält und ein Krankheitsantigen codiert, das von einem Krankheitserreger stammt oder ein Krebsantigen oder eine modifizierte Form davon ist, dem aber die codierende Sequenz für eines oder mehrere retrovirale Proteine fehlt, und wobei die Zelle auch virale Proteine zur Produktion des rekombinanten Retrovirus exprimieren kann.

10. Zellen nach Anspruch 8 oder 9 zur Verwendung in einem Verfahren zur Behandlung des menschlichen oder tierischen Körpers.

## Revendications

1. Un rétrovirus recombinant incapable de se répliquer, capable d'infecter des cellules humaines, portant une construction de vecteur capable de prévenir, d'inhiber, de stabiliser ou de renverser le cours de maladies cancéreuses ou auto-immunes,
dans lequel la construction de vecteur commande l'expression d'un antigène caractéristique de maladie qui provient d'un agent pathogène ou est un antigène cancéreux ou une forme modifiée de ceux-ci dans des cellules cibles infectées par le rétrovirus,
ledit antigène ou ladite forme modifiée de celui-ci étant capable de stimuler une réponse immunitaire à médiation cellulaire chez l'homme.

2. Un rétrovirus recombinant selon la revendication 1 dans lequel la construction de vecteur commande l'expression d'une forme modifiée d'un antigène caractéristique de maladie qui provient d'un agent pathogène dans des cellules cibles infectées par le rétrovirus, ledit antigène modifié étant capable de stimuler une réponse immunitaire chez un homme mais dont la pathogénicité est réduite par rapport à l'agent pathogène.

3. Un rétrovirus recombinant selon la revendication 1 ou 2 dans lequel l'antigène exprimé induit une réponse immunitaire restreinte par les HLA de classe I ou II.

4. Un rétrovirus recombinant selon l'une quelconque des revendications 1 à 3 dans lequel l'antigène exprimé est une protéine de HIV sélectionnée dans le groupe constitué de gp160, gp120 et gp41, ou des formes modifiées de celles-ci.

5. Une composition pharmaceutique comprenant un rétrovirus recombinant selon l'une quelconque des revendications 1 à 4 en combinaison avec un véhicule ou un diluant physiologiquement acceptable.

6. Un rétrovirus recombinant selon l'une quelconque des revendications 1 à 4 ou une composition selon la revendication 5 destiné à une utilisation comme substance thérapeutiquement active dans une méthode de traitement du corps humain.

7. Méthode de production d'un rétrovirus recombinant tel que défini selon l'une quelconque des revendications 1 à 4 comprenant :
l'encapsidation d'une construction de vecteur dans une capside et une enveloppe de façon que ledit rétrovirus soit produit.

8. Cellules ex vivo infectées par un rétrovirus recombinant selon l'une quelconque des revendications 1 à 4.

9. Cellule eucaryote capable de produire un rétrovirus recombinant incapable de se répliquer selon l'une quelconque des revendications 1 à 4, ladite cellule contenant un génome rétroviral dudit rétrovirus recombinant ayant un signal d'encapsidation et codant pour un antigène caractéristique de maladie qui provient d'un agent pathogène ou est un antigène cancéreux ou une forme modifiée de ceux-ci mais dépourvu de la séquence codant pour une ou plusieurs protéines rétrovirales et ladite cellule étant également capable d'exprimer des protéines virales pour la production dudit rétrovirus recombinant.

10. Cellules selon la revendication 8 ou la revendication 9 destinées à une utilisation dans une méthode de traitement du corps humain ou animal.
